# EUROPEAN PATENT APPLICATION

(11) **EP 4 804 197 A1**
(43) Date of publication of application: **09.09.2026**
(21) Application number: 25161928.4
(22) Date of filing: 05.03.2025
(51) Int. Cl.: G16H 10/20, G16H 20/00, G16H 50/20, G16H 80/00, G16H 70/20

(54) **SYSTEM AND METHOD FOR SUPPORTING INFORMED CLINICAL DECISIONS IN HEALTHCARE**

(71) Applicant: Dear Health Netherlands B.V., 2333 CW Leiden (NL)
(72) Inventor: van der Meulen, Alson Leendert Hoop, 2333 CW Leiden (NL); de Noo, Job, 2333 CW Leiden (NL)
(74) Representative: DTS Patent- und Rechtsanwälte PartmbB

(57) **Abstract**

The present invention provides a system and method for supporting healthcare professionals in making informed clinical decisions, wherein the system comprises an event management system configured to transform patient data into a uniform format, wherein the patient data comprises input data received from a patient, medical data received from healthcare professionals and/or from a health information system, and historical patient data stored in the system; and a recommendation module configured to generate a recommendation for the patient in response to the transformed patient data received from the event management system.

## Description

The present invention relates to clinical decision support systems, and specifically to a system and method for supporting healthcare professionals in making informed clinical decisions.

Existing clinical decision support systems (CDSS) play an important role in assisting healthcare practitioners by providing recommendations based on a combination of patient data and clinical guidelines. However, the functionality of most current CDSS is significantly limited due to their reliance on a limited set of inputs and static triggers. For instance, many systems base their recommendations solely on pre-defined workflows, such as questionnaires completed by physicians or data extracted from electronic medical records (EMR). While these inputs are useful, they fail to capture the dynamic nature of healthcare scenarios where critical events or updates occur outside the context of hospital automation systems.

For example, a patient may report symptoms, adverse reactions, or other health-related events directly through digital tools or over the phone, but such information is often excluded from the decision-making process of traditional CDSS. These systems lack the capability to combine patient-reported data with EMR data and/or other practitioner-supplied information to create a complete and up-to-date view of the patient's condition. As a result, they are less adaptable to the unpredictable and evolving needs of patient care.

Additionally, most existing CDSS operate in an on-demand way. These systems are reactive to the system users. For example, the existing systems must be invoked by the practitioner's system to retrieve recommendations, rather than automatically responding to events as they occur. The on-demand mode of operation causes delays in response times and adds extra work for healthcare providers. The existing systems are reactive, and practitioners might not receive timely alerts or actionable recommendations unless they specifically seek them out. This reactive nature reduces the likelihood of practitioners being continuously aware of emerging risks or critical opportunities to intervene in patient care.

It is therefore an object of the present invention to address the limitations of these systems. In particular, the present invention aims to provide a more dynamic, event-driven clinical decision support system.

This object is achieved in the present invention by a system and method for supporting healthcare professionals in making informed clinical decisions according to the independent claims.

The system comprises:
- an event management system configured to transform patient data into a uniform format, wherein the patient data comprises input data received from a patient, medical data received from healthcare professionals and/or from electronic health records, and historical patient data stored in the system; and
- a recommendation module configured to generate a recommendation for the patient in response to the transformed patient data received from the event management system.

The invention is based on the basic idea to provide a responsive system that is configured to automatically generate personalized care recommendations in response to updates and/or changes in patient data. The system comprises a data event management system that is configured to transform the patient data into a uniform data format. The system further comprises a recommendation module that is configured to transform real-time patient input and relevant clinical data into highly personalized care recommendations. The decision-support process is fully transparent and enables health care providers to deliver the best possible care tailored to each patient. Advantageously, the present invention provides a system that is configured to function proactively and respond automatically to events as they occur by generating personalized recommendations.

In particular, the data event management system serves as a data event extractor. It is configured to receive events (e.g. from microservices via an event bus). The data event management system is further configured to determine if the incoming event type can be mapped to a data event. If so, it transforms the event into a uniform format.

The event-driven recommender (e.g. a recommendation module) listens to these data events on the event bus, retrieves relevant rules for the healthcare provider from a medical content management system, and evaluates these rules against the incoming data and historical patient data. If this evaluation generates a recommendation or smart notification, the recommender stores it for access through the practitioner portal.

Advantageously, the shift from on-demand to event-driven decision support would not only improve response times but also enhance the overall quality and timeliness of care, ultimately leading to better patient outcomes.

In particular, the system according to the present invention is configured to integrate diverse inputs-including patient-reported events, EMR data, and practitioner-supplied observations-would enable more responsive and effective recommendations

In particular, the system is further configured to display the recommendation generated by the recommendation module to the healthcare professionals, preferably through a user interface platform.

In particular, the recommendation generated by the recommendation module is integrated into the historical patient data.

In particular, the recommendation module, preferably an event-driven recommendation module, comprises a recommender engine configured to generate a recommendation based on data events generated by the event management system, wherein the data events are in a uniform format regardless of triggering events.

In particular, the events comprise event-specific data such as updates to medical records, changes in patient conditions and inputs received through the user interface platform and/or a medical content management system.

In particular, the system is configured to store the patient data in the form of events (occurring in the system), wherein the events comprise event-specific data in heterogenous formats.

In particular, the event management system comprises a transformation module configured to transform the events into corresponding data events, wherein the data events comprise a homogenous (uniform) format.

In particular, the historical patient data comprises records of input data, medical data, previous events and a patient profile. The historical patent data includes a history of past events and data events.

In particular, the historical patient data refers to previously recorded information (events) that provides a comprehensive account of a patient's health-related activities and conditions over time.

In particular, the historical patient data comprises patient input data, such as patient-reported symptoms or responses to questionnaires; medical information, such as diagnoses, treatments, and medication history; previous events; and/or a patient profile, which combines key attributes and trends related to the patient's overall health status.

In particular, the patient profile further comprises recommender keys. The recommender keys represent a history of medical records and patient-specific information.

In particular, the transformation module is configured to determine whether each of the events can be mapped to (or associated with) a predefined data event, upon determining that the events can be mapped to the corresponding data events, to transform (or convert) the data events into a standard format, e.g. a flat key-value pair format, which is included in the corresponding data event.

In this way, the flat key-value pair format is stored within the data event to standardize the representation of the event for machine-readable storage, analysis, or further processing.

In particular, the event management system further comprises an event bus configured to communicate the events to a transformation module.

In particular, the event management system further comprises a data event bus configured to publish the data events received from the transformation module.

In particular, the transformation module is configured to receive one or more events from an event bus.

In particular, the recommendation module, preferably an event-driven recommendation module, comprises a recommender engine, configured to generate recommendations based on the data events generated by the event management system.

In particular, the recommender engine is configured to receive the data events, preferably through a data event bus, to retrieve clinical rules, preferably from a medical content management system, and to evaluate the clinical rules against the received data events (including historical patient data compiled from past events).

In particular, the recommendation module is configured to invoke an external artificial intelligence model, and to generate a recommendation based on the outcome of the artificial intelligence model.

In particular, the external AI model processes the patient data and returns a recommendation related to the patient's care.

For example, the external AI model is hosted by a third party, e.g. on a cloud-based platform, and is accessible by the recommendation module through a network.

In particular, the recommendation module is further configured to generate and store recommendations and smart notifications based on the evaluation of the rules and the data, for subsequent access through the user interface platform.

In particular, the system further comprises a user interface platform, preferably a practitioner portal, configured to enable healthcare professionals to access patient information, decision-making tools, and/or patient clinical data.

In particular, the system further comprises a medical content management system comprises content types describing structured data. The medical content management system is configured to store the medical content in a structured format based on the defined content types, preferably types of the medical contents comprising at least one of questionnaires, recommendations, and clinical rules.

For example, software engineers create content types in the content management system, such as a questionnaire content type. Medical engineers then create content using those types, like a questionnaire for ovarian cancer, a questionnaire for IBD, etc. Then this content is used by a modular service module for a user interface platform and a patient interface. For example, the patient is asked to fill in the questionnaire created by the medical engineer. And the responses to this questionnaire are stored in the modular service module.

For example, the stored medical contents are configured to be extracted in a machine-readable format, e.g. by a medical engineer.

In particular, the system further comprises a patient interface, preferably a software application, configured to facilitate the patient to interact with the system, preferably to access the health records, schedule appointments, and communicate with the healthcare professionals.

In particular, the system further comprises a modular service module, preferably microservices, configured to communicate the patient data received from a user interface platform and/or the patient interface to the event management system, preferably to the transformation module.

In particular, the modular service module comprises independent (smaller) independent services, each responsible for a specific function.

The independent services are configured to communicate with each other through events and/or well-defined APIs (Application Programming Interfaces). This, in turn, results in flexibility, scalability, and ease of maintenance.

For example, microservices are configured to handle patient input.

In particular, the interactive interface is configured to enable the medical engineers to enter, view, and edit the structured medical contents stored in the medical content management system (medical CMS) using electronic forms.

In particular, the interactive interface is used by medical engineers to manage medical content, like questionnaires, recommendations and recommender rules. For example, the CMS contains a number of content types, like questionnaire or recommendation. The content types are managed by software engineers, and the medical contents by medical engineers.

In particular, the system, e.g. the medical CMS, comprises a data storage module (e.g. a database) configured to store the structured data.

In particular, the system, e.g. the medical CMS, further comprises a data extraction module configured to extract the stored medical contents in a machine-readable format for further use in the modular service module.

In particular, the medical CMS comprises recommender rules for the recommendation module.

In particular, the medical CMS is configured to communicate with a user interface platform and a recommendation module.

In particular, the system further comprises a data management module, preferably data services, configured to aggregate, process, and distribute the patient data across the system.

In particular, the data management module is configured to communicate with the transformation module and to receive the data events from a data event bus.

Advantageously, the data management module ensures that data is accessible, reliable, and usable across various system components and applications. The data management module configured for handling the storage, retrieval, and management of data in the system.

In particular, the system further comprises an interactive interface for medical users e.g. medical engineers, for accessing and processing the patient data, for example through forms that correspond to the structured format of each content type.

In particular, the interactive interface is configured to:
- communicate with the medical content management system (122), and/or
- create configurable clinical rules, wherein the rules define how to respond to different types of the patient data (or events), and/or
- create or update recommendation definitions, and/or
- create or update questionnaires.

In particular, the system further comprises a synchronization control module, preferably a fast healthcare interoperability module, configured to synchronize patient data (e.g. input data and/or medical data), to enable information exchange within the system, wherein the synchronization control module is in communication with a health information system.

In particular, the synchronization control module (e.g. a data interoperability module) is configured to enable exchange of information between different entities in the system, e.g. between the health information system, the modular service management and the event management system.

In particular, the synchronization control module can be further configured to ensure real-time updates to patient records and communication of changes to the user interface platform and the patient interface.

In particular, the system further comprises a cloud-based storage system, preferably comprising a user interface platform, a medical content management system, a modular service module, an event management module, a data management module and a decision support module

In particular, the system includes a data event extractor, a recommender engine, and a practitioner portal, each playing a role in improving patient care.

The data event extractor transforms data from patients, practitioners, and electronic health records into a uniform format. The recommender engine uses these transformed events, along with historical patient data compiled from previous events, to evaluate clinical rules and provide personalized recommendations. Finally, the practitioner portal displays these recommendations, allowing healthcare providers to review, approve, and execute them.

This integrated system enables better recommendations and smart notifications for doctors, nurses, and patients. This in turn improves patient wellness and outcomes. The system of the present invention also reduces healthcare expenses and minimizes the risk of human errors.

In particular, the system comprises a module that receives various patient data (raw data), transforms them into events and converts the events into data events, wherein the (new) data events are all in the same, standardized format. Advantageously, this uniformity enhances the capability and accuracy of the system.

In particular, the event driven recommender is embedded within a cloud-based platform. The event driven recommender alternatively is configured to be used within any event driven architecture.

In particular, when the system receives data, this data is stored as an event. When an event happens (e.g. a patient fills in a questionnaire, a new lab result comes in, hospital staff schedules a new appointment, etc.), the system initiates a sequence of operations:
Event Reception: The system receives the incoming event through a patient app, a practitioner portal or an integration like with Electronic Medical Records. This event contains event-specific data (e.g., lab test values, questionnaire answers).

Data Extraction/Transformation: Both the incoming event data and the patient's historical medical data are passed through a data event extractor, which extracts relevant (not personally identifiable) data and transforms them to a flat set of standardized key-value pairs. This transformation process ensures uniformity. in how data is processed and allows for easier rule application.

Rule Execution: Once data is transformed, the system accesses a set of configurable rules established by medical engineers. These rules define how to respond to different types of events. The rules can consider both the event data and the historical data of the patient, allowing for personalized medical recommendations.

The rules may include logic such as:
- Triggering alerts if lab results fall outside a certain range.
- Suggesting follow-up tests based on past medical history combined with new event data.

Historical Data Update: After processing the event, the system appends the new event information to the patient's historical data, ensuring that future recommendations will be informed by the most recent updates in the patient's health status.

Advantageously, the event-driven recommender system is highly flexible and scalable. New events can be added, and rules can be updated or extended by medical engineers without requiring system-wide changes, making it adaptable to evolving medical needs and protocols.

The present invention further relates to a method of supporting healthcare professionals, in particular using a system.

The method comprises the steps of:
- transforming, using an event management system, patient data into a uniform format, wherein the patient data comprises input data received from a patient, and/or medical data obtained from healthcare professionals and electronic health records; and
- (automatically) generating, using a transformation module, a recommendation in response to receiving the transformed patient data.

In particular, the method further comprises the step of reviewing the recommendation, preferably on a user interface platform, where the healthcare professionals either approve or disapprove the recommendation.

In particular, the method further comprises the step of storing the approval / disapproval of the healthcare professional in a modular service module serving as input for the medical engineer to update the rules.

In particular, the method further comprises the step of updating medical data within the modular service module and/or the health information system to include the approved or disapproved recommendation.

For example, depending on the type of recommendation, the method comprises updating medical data within the modular service module 128, preferably changing the treatment plan for the patient, and/or updating medical data within the health information system to include the approved or disapproved recommendation.

In particular, a recommendation comprises a variety of actions, such as assigning a new treatment plan, modifying an existing one, displaying a message to a physician or the healthcare professionals, or sending a message to a patient, or the like.

In particular, when the recommendation is accepted, an action is typically carried out, or the system recognizes it as confirmation that the physician has followed the recommended course of action (such as prescribing medication). If the recommendation is rejected (disapproved), no immediate action is taken by the system, but the rejection is recorded for future optimization of the recommendation rules.

It is schematically shown in
- **Fig. 1**: a block diagram of a system according to the present invention;
- **Fig. 2**: an example of rules developed base of the clinical guidelines.
- **Fig. 3**: a block diagram of an event management system;
- **Fig. 4**: a block diagram of a method for supporting healthcare professionals according to the present invention;
- **Fig. 5**: a block diagram illustrating the execution of the method using system;
- **Fig. 6**: a block diagram of an exemplary workflow illustrating how the system responses to a patient data input; and
- **Fig. 7**: a flowchart of series of actions executed through interaction with the system.

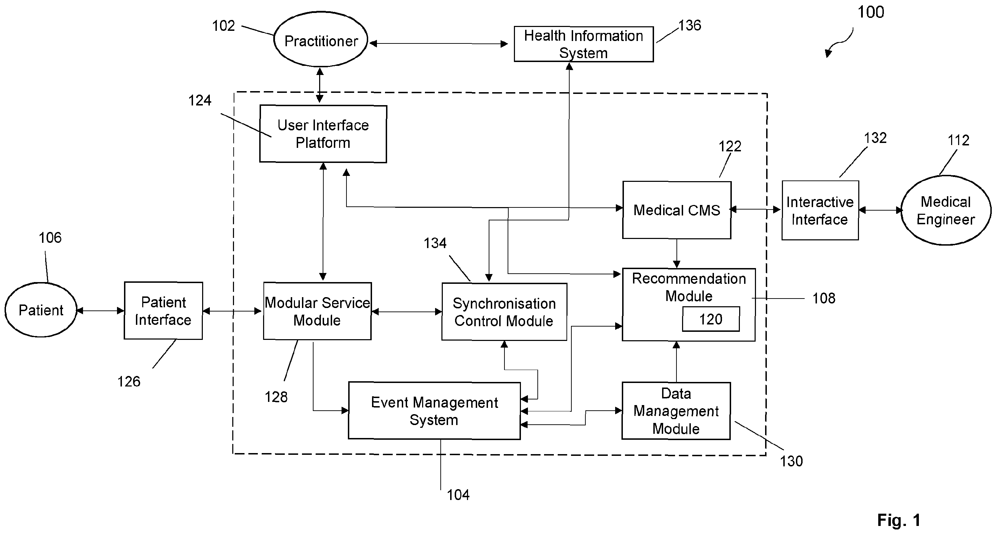

Fig. 1 shows a block diagram of a system 100 for supporting healthcare professionals 102 in making informed clinical decisions according to the present invention.

The system 100, for example, is a clinical decision support system.

The system 100 comprises an event management system 104 that is configured to transform patient data into a uniform format.

The patient data comprises, for example, input data received from a patient 106.

The patient data further comprises medical data received from healthcare professionals 102 and/or electronic health records.

The patient data further comprises historical patient data, for example, past input data and past medical data, that have already been stored in the system.

The received patient data, which are the single source of truth, are stored, for example, in a central repository or a global event store.

The system 100 is configured to store, e.g. in a storage module, the patient data (e.g. the patient input data, the medical data and the historical patient data) in the form of events occurring in the system.

In particular, an event comprises an event-specific data, representing a discrete piece of information or occurrence, such as a measurement, observation, or recorded activity.

The events therefore comprise heterogenous formats.

The system 100 is further configured to store patient profiles, e.g. in the storage module.

Alternatively, cloud-based storage solutions, such as AWS S3, are commonly employed to securely and scalably store the profiles and associated metadata.

The patient profiles further comprise a history of various properties and values, referred to as "recommender keys."

The recommender keys represent specific patient properties or attributes that are relevant for medical decision-making. For example, a recommender key represents a property like a vital sign (weight, blood pressure), lab value (blood glucose level), observation (tumor stage determined by a doctor), etc.

The event management system 104 is advantageously configured to extract information from a variety of events based on actions by the patient 106, actions by the health professional (practitioner) 102 and updates happening within the electronic health records.

For example, a transformation module 114 of the event management system 104 is configured to determine (or check) whether each of the events can be mapped to (or associated with) a predefined data event.

For example, the predefined data events are stored in the event management system 104.

Upon determining that the events can be mapped to the corresponding data events, the transformation module 114 is configured to transform (or convert) the data events into a standard or uniform format, e.g. a flat key-value pair format, in the corresponding data event.

The transformed format is uniform, regardless of the event format that triggers the transformation.

For example, in the flat key-value format data are stored in pairs, where each key is unique and maps to a single value.

The system 100 further comprises a recommendation module 108 that is configured to generate a recommendation for the patient based on the transformed data.

The recommendation module 108 further comprises the storage module configured to store the events and the data events produced in the transformation module 114.

The recommendation module 108 functions as an event-based recommender that will respond to data events produced by the event management system, and/or evaluate clinical rules written by medical experts based on literature and guidelines, and/or store a history of events for future reference.

The clinical rules are defined based on clinical guidelines and are expressed in a logical, structured format.

The clinical rules comprise logical statements that allow the system to automatically generate recommendations based on user inputs, reducing the need for manual decision-making. The rules typically take the form of "if ... then" statements, e.g. "If the patient's blood pressure exceeds a threshold, then recommend medication X".

Clinical rules may be used for risk assessment, applying scientific / clinical guidelines or supporting existing clinical processes.

Medical engineers further develop rules to automate these processes and automatically update it every time one of the parameters, like lab values, changes.

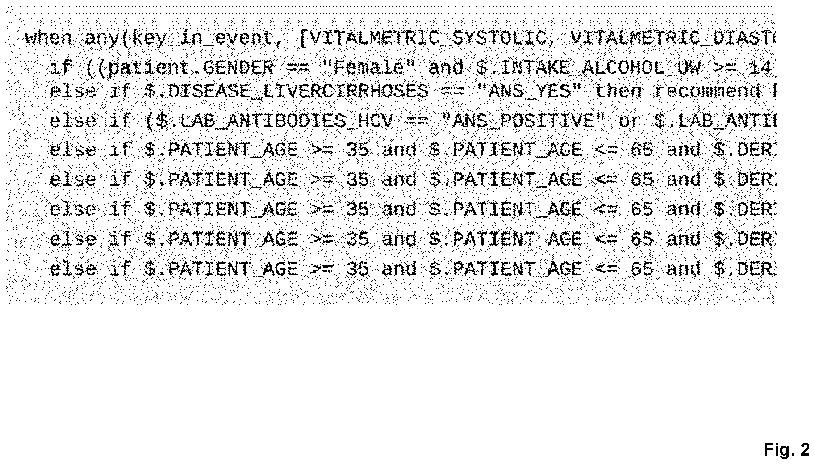

Fig. 2 shows an example of the clinical rules developed on the basis of the clinical guidelines.

The rules or guidelines serves as additional external knowledge to support personalized recommendations.

For example, the rules are integrated into the system 100, e.g. into the recommendation module 108.

The rules are configured to be extracted from a medical content management system (medical CMS). The rules are further configured to be stored in the recommendation module, which provides an event-based recommender service.

In particular, the system 100 is further configured to display the recommendation generated by the recommendation module 108 to the healthcare professionals 102, for example, through a user interface platform 124.

The system 100 further comprises a medical content management system 122.

In particular, the medical content management system 122 is configured to define various content types describing medical content. Examples of medical content types are questionnaires, recommendation definitions and recommender rules.

For example, a content type for questionnaires includes fields like question text, options for answers, and patient demographics. A recommendation content type might include structured fields for the medication name, dosage, and patient conditions.

The such structured data are configured to be extracted in a machine-readable format, e.g. by a medical engineer 112 using an interactive interface 132. The machine-readable data makes it easy to automate workflows and analyze data.

The recommendations generated by the recommendation module 104 comprise pieces of advice or instructions (e.g., medical prescriptions or treatment plans). An example could be "Prescribe medication X to patient Y". The recommendations are structured and can be based on data inputs from the medical professional, such as patient condition, medical history, and symptoms.

Advantageously, this structured approach ensures that data can be easily stored, retrieved, and efficiently used.

The interactive interface 132 advantageously allows medical engineers (or clinicians) to interact with the system and enter, view, and edit data in the structured format.

The system 100, e.g. the medical content management system 122, can be used to configure patient profiles that display data from the data events.

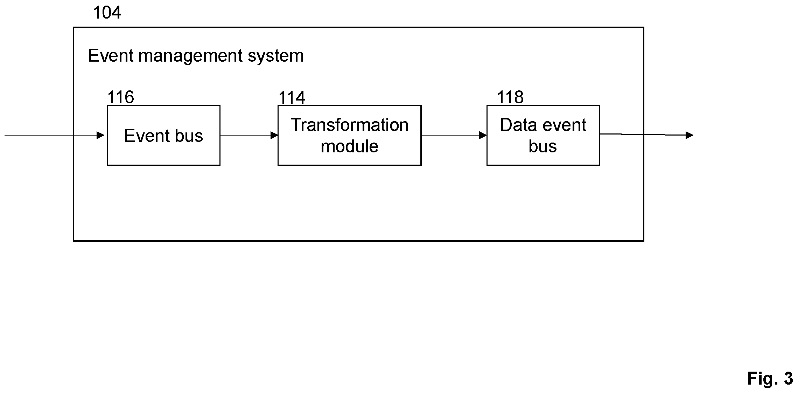

In Fig. 3 a block diagram of the event management system 104 is shown.

The event management system 104 comprises a transformation module 114 that is configured to transform the events into corresponding data events.

From the events, the transformation module 114 is configured to derive intermediate data structures in a homogenous format.

In particular, the transformation module 114 is configured to process the events to produce (structured) data events in a uniform format. Advantageously, the data events remain consistent in format and are easier to analyze and organize.

The data events comprise a homogenous (uniform) format.

The transformation module 114 functions as a data event extractor.

The event management system 104 further comprises an event bus 116 that is configured to communicate (e.g. receive and transmit) the events to the transformation module 114.

The event management system 104 further comprises a data event bus 118 that is configured to publish the data events received from the transformation module 114 (see Fig. 3).

The recommendation module 108 comprises a recommender engine 120 that is configured to generate recommendations based on the data events received from the event management system 104.

The recommender engine 120 is configured to receive the data events through a data event bus 118, and to retrieve clinical rules, for example from a medical content management system 122.

The recommender engine 120 is further configured to evaluate the clinical rules against the received data events.

The recommendation module 108 is further configured to invoke an external artificial intelligence (AI) model, and to generate a recommendation based on the outcome of the AI model.

The system 100 further comprises a user interface platform 124, such as a practitioner portal.

The user interface platform 124 is configured to enable healthcare professionals 102 to access patient information, decision-making tools, and/or patient clinical data.

The system 100 further comprises a patient interface 126, preferably a software application, configured to facilitate the (authorized) patient 106 to interact with the system 100-

For example, the patient 106 can use the patient interface 126 to access their health records, schedule appointments, and communicate with the healthcare professionals 102.

The system 100 further comprises a modular service module 128, preferably microservices, that is configured to communicate the patient data received from a user interface platform 124 and/or the patient interface 126 to the transformation module 114 of the event management system 104.

The patient data is configured to be structured and stored using the modular service module 128 according to specific schemas, e.g. tables, fields, or attributes. For example, the patient data is configured to be stored in a storage module with clear fields for name, age, medical history, etc.

The system 100 further comprises a data management module 130, preferably data services, that is configured to aggregate, process, and distribute the patient data across the system 100.

The data management module 130 is configured to communicate with the transformation module 114 and to receive the data events from a data event bus 118.

The system 100 further comprises an interactive interface 132 for medical users, e.g. medical engineers, for accessing and processing the patient data, for example through forms that correspond to the structured format of each content type.

The interactive interface 132 is configured to communicate with the medical content management system 122.

The interactive interface 132 is configured to create configurable clinical rules, wherein the rules define how to respond to different types of the patient data (or events).

The system 100 further comprises a synchronization control module 134, preferably a fast healthcare interoperability module.

The synchronization module 134 is configured to synchronize data (e.g. patient data) enable information exchange within the system 100, wherein the synchronization control module 134 is in communication with the health information system 136.

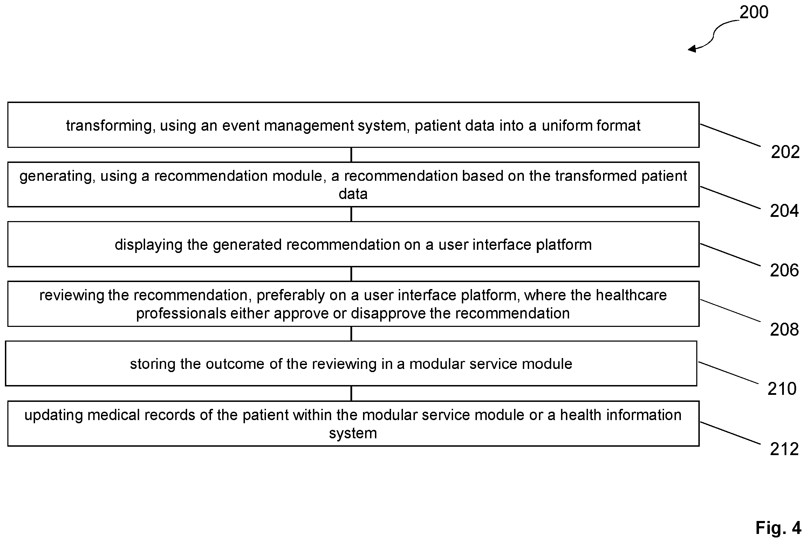

Fig. 4 shows a flowchart of a method 200 for supporting healthcare professionals 102, for example, using a system 100.

The method 200 comprises the steps of:
- transforming 202, using an event management system 104, patient data into a uniform format; and
- generating (204), using a recommendation module 108, a recommendation in response to receiving the transformed patient data from the event management system 104.

The patient data comprises input data received from a patient 106, and/or medical data obtained from healthcare professionals 102 and electronic health records

The method 200 further comprises the step of displaying 206 the generated recommendation on a user interface platform 124.

The method 200 further comprises reviewing 208 the recommendation, preferably on a user interface platform 124, where the healthcare professionals 102 either approve or disapprove the recommendation.

The method 200 further comprises storing 210 the outcome of the review 208 by the healthcare professional 102 in the modular service module 128 for future use by the medical engineer to update the rules, for example if the recommendation of a rule is very often rejected, the rule might need updating.

The method 200 further comprises, updating 212 the patient's medical data within the modular service module 128 (for example updating a treatment plan) or the health information system 136 (for example entering a prescription).

In particular, the patient data is provided through one or more of the following steps:
- receiving, preferably using a patient interface 126, input data from a patient 106;
- receiving, preferably using a user interface platform 124, medical data from healthcare professionals 102 and/or medical users;
- receiving, preferably using a synchronization control module 134, medical data from a health information system 136
- receiving medical data from a third-party system through a custom direct API.

In particular, the method 200 further comprises aggregating, preferably using a data management module 130, the input data and the medical data.

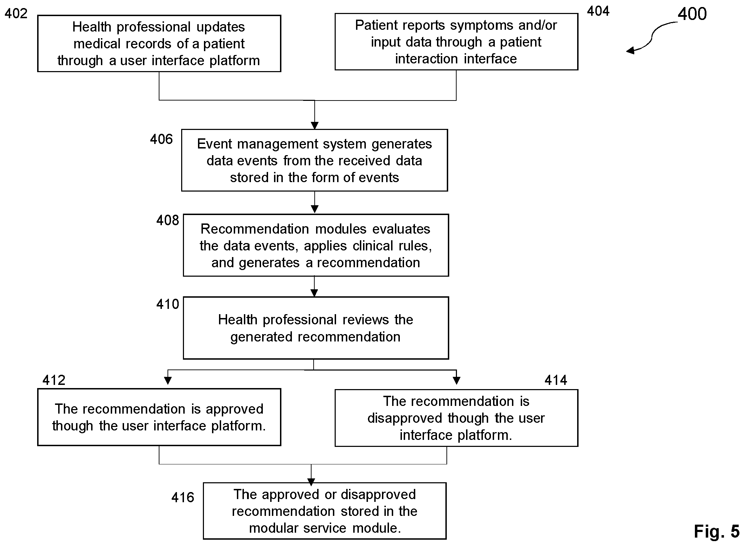

Fig. 5 shows a block diagram illustrating the execution of method 200 using system 100.

At block 402, the health professional updates medical records through the user interface platform 124.

At block 404, patient reports symptoms and/or input data through the patient interface 126.

At block 406, the event management system 104 generates data events from the received data stored in the form of events.

At block 408, the recommendation module 108 evaluates the data events, applies clinical rules, and generates a recommendation.

At block 410, the health professional reviews the generated recommendation

At blocks 412, 414, the recommendation is approved or disapproved though the user interface platform 124.

At block 416, the approved or disapproved recommendation is stored in the modular service module 128 and the outcome may be entered in a health information system 136.

For example, the outcome of reviewing the recommendations, such as a new treatment plan, nutritional advice, or a new prescription, may be stored in the modular service module (for treatment plans), the health information system (for prescriptions), and eventually, treatment plans may also be entered into the health information system 136.

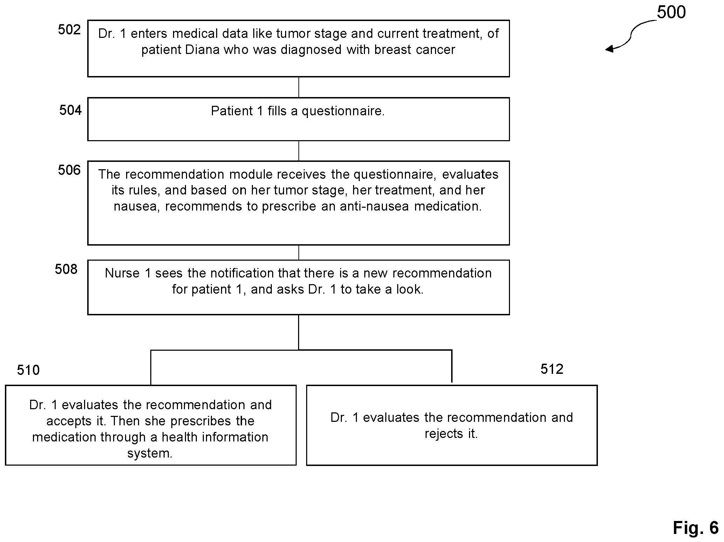

Fig. 6 shows an exemplary workflow for using the system 100.

In this example, the system 100 reacts on a patient-reported outcome.

At block 502, the treating health professional 102 enters the medical data and current treatment of a patient into the user interface platform 124.

The patient, for example, is patient 1 who was diagnosed with breast cancer. The medical data, for example, comprises the stage of tumor or lab results.

At block 504, the patient interacts with the system, preferably via the patient interface 126.

The patient reports about her or his current health status and the symptoms.

In this example, patient 1 fills in a questionnaire reporting suffering from nausea.

At block 506, the recommendation module 108 receives the questionnaire, and applies the clinical rules to evaluate the questionnaire.

The recommendation module 108 then recommends to prescribe anti-nausea medication based on the patient's historical data, for example, the tumor stage, the treatment plan of the patient and the reported symptom (nausea).

The produced recommendation can now be reviewed by the health professional 102 (e.g. Doctor 1).

At block 508, the nurse, nurse 1, sees the notification that there is a new recommendation for patient 1.

The nurse may inform the health professional 102 to review the new recommendation. Alternatively, the health professionals 102 themselves see the notification in the practitioner portal and review the new recommendation.

At block 510, the health professional 102 (Dr. 1) evaluates the new recommendation and accepts it. The health professional 102 then takes actions, e.g. prescribe the medication through the health information system 136 (e.g. an electronic medical record).

Alternatively, at block 512, the health professional 102 rejects the new recommendation. In this case, the nurse may contact, via application or a telephone call, the patient to advice how she can change her diet to reduce her nausea.

Another exemplary workflow (not shown) using the system 100 involves, for example, a lab test for kidney function prompting a doctor to lower a patient's medication dose based on guidelines.

In this case, the historical data of patient 106 shows that the patient suffers from rheumatoid arthritis and has a kidney function with an eGFR of 85 ml/min, which is acceptable. The patient takes 25 mg of Methotrexate per week.

The medical data of patient 106 in the health information system 136 includes a lab test for kidney function, recording an eGFR of 40 ml/min, which indicates impaired kidney function.

The recommendation module 108 receives the lab records through the synchronization control module 134 and evaluates its clinical rules. Based on guidelines, the recommendation module adjusts the patient's medication by reducing the Methotrexate dose to 12.5 mg per week.

Subsequently, the nurse is notified of the new recommendation for patient 106 on the user interface platform 124 and informs the health professional 102 to review it. Alternatively, the health professional 102 directly receives a notification about the new recommendation on the user interface platform.

The health professional 102 evaluates the new recommendation and accepts it. The health professional 102 then updates the medication dosage in the health information system 136 (e.g., the electronic medical record).

Alternatively, the health professional 102 may reject the new recommendation and instead switch the patient to a different medication.

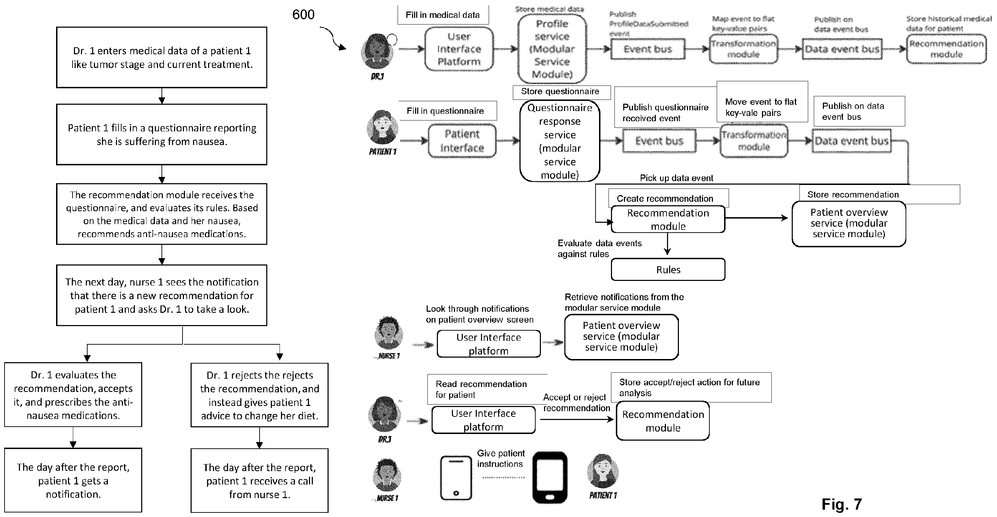

Fig. 7 illustrates a flowchart 600 of a series of actions executed in the system 100 in response to user interaction.

In this figure, a block diagram of an example explained in view of Fig. 6 is also shown on the left-side of Fig. 7.

The health professional, Dr. 1, interacts with the system, e.g., by entering medical data of a patient 1, updating or generating the patient profile through the entry of medical data in the user interface platform 124. The medical data is then transferred via a profile service (part of the modular service module 128) to the event bus 116. For example, the medical data includes tumor stat and current treatment of the patient 1 who was diagnosed with breast cancer.

The transformation module 114 transforms the medical data to a uniform data event and transfers it to the data event bus 118, where it is forwarded to the recommendation module 108.

Similarly, the patient 1 interacts with the system by 100 completing a questionnaire, e.g. via the patient interface 126, that she is suffering from nausea. The questionnaire data is sent to the event bus 116 via a questionnaire response service (a microservice part of the modular service module 128).

The transformation module 114 receives the event and transforms it to a data event, which is then published on the data event bus 118.

The recommendation module 108 (e.g. functions as an event-based recommender) receives the data event and applies its rules and considers the historical patient data to generate a recommendation.

In this example, the recommendation module 108 receives the questionnaire and evaluates its rules. Based on the tumor stage, treatment of patient 1, and her nausea, it recommends prescribing anti-nausea medications.

The patient overview service (part of the modular service module 128) is updated with a notification that a new recommendation is available for the patient.

The recommendation is observed by nurse 1 through the user interface platform 124 in the patient overview service, and the practitioner is notified to review the recommendation.

This automatically generated recommendation is then reviewed by Dr. 1 through the user interface platform 124, who either accepts or rejects it. If accepted, she prescribes the necessary medication; if rejected, she may, for example, suggests an alternative diet.

In this example, the Dr. 1 evaluates the recommendation and accepts it. Dr. 1 then prescribes the anti-nausea medication through the health information system 136 (e.g. electronic medical record). Alternatively, Dr. 1 rejects the rejects the recommendation, and instead gives patient 1 advice to change her diet.

The patient is then informed accordingly. In this example, the day after patient 1 reported nausea, she gets a message from her pharmacy that her anti-nausea medication is available for pick-up. Alternatively, the patient receives a call from Nurse 1 with advice on how to change her diet to reduce nausea.

### REFERENCE NUMERALS

- 100: system
- 102: healthcare professionals
- 104: event management system
- 106: patient
- 108: recommendation module
- 110: user interface platform
- 112: medical engineer
- 114: transformation module
- 116: event bus
- 118: data event bus
- 120: recommender engine
- 122: medical content management system
- 124: user interface platform
- 126: patient interface
- 128: modular service module
- 130: data management module
- 132: interactive interface
- 134: synchronization control module
- 136: health information system

- 200: method
- 202 - 212: method steps

- 400: block diagram
- 402-416: blocks

- 500: block diagram
- 502-512: blocks

- 600: flowchart

## Claims

1. A system (100), in particular a clinical decision support system, for supporting healthcare professionals (102) in making informed clinical decisions, wherein the system (100) comprises:
- an event management system (104) configured to transform patient data into a uniform format, wherein the patient data comprises input data received from a patient (106), medical data received from healthcare professionals (102) and/or from a health information system (136), and historical patient data stored in the system; and
- a recommendation module (108) configured to generate a recommendation for treatment of the patient in response to the transformed patient data received from the event management system.

2. The system (100) according to claim 1,
**characterized in that**
the system (100) is further configured to display the recommendation generated by the recommendation module (108) to the healthcare professionals (102), preferably through a user interface platform (124).

3. The system (100) according to claim 1 or claim 2,
**characterized in that**
the system (100) is configured to store the patient data in the form of events (occurring in the system), wherein the events comprise event-specific data in heterogenous formats,
the event management system (104) comprises a transformation module (114) configured to transform the events into corresponding data events, wherein the data events comprise a homogenous format.

4. The system (100) according to claim 3,
**characterized in that**
the transformation module (114) is configured to:
- determine whether each of the events can be mapped to (or associated with) a predefined data event,
- upon determining that the events can be mapped to the corresponding data events, transform the data events into a standard format in the corresponding data event.

5. The system (100) according to claim 3 or claim 4,
**characterized in that**
the event management system (104) further comprises an event bus (116) configured to communicate the events to a transformation module (114), and/or the event management system (104) further comprises a data event bus (118) configured to publish the data events received from the transformation module (114).

6. The system (100) according to one of claims 3 to 5,
**characterized in that**
the recommendation module (108), preferably an event-driven recommendation module, comprises a recommender engine (120), configured to generate recommendations based on the data events generated by the event management system (104).

7. The system (100) according to claim 6,
**characterized in that**
the recommender engine (120) is configured to:
- receive the data events, preferably through a data event bus (118),
- to retrieve clinical rules, preferably from a medical content management system (122), and
- evaluate the clinical rules against the received data events.

8. The system (100) according to one of the preceding claims,
**characterized in that**
the recommendation module (108) is configured to invoke an external artificial intelligence model, and to generate a recommendation based on the outcome of the external model.

9. The system (100) according to one of the preceding claims,
**characterized in that**
- a user interface platform (124), preferably a practitioner portal, configured to enable healthcare professionals (102) to access patient information, decision-making tools, and/or patient clinical data; and/or
- a medical content management system (122) configured to store medical contents in a structured format based on the predefined content types, preferably the content types comprising a questionnaire content type, recommendation definitions, and clinical rules; and/or
- a patient interface (126), preferably a software application, configured to facilitate the patient (106) to interact with the system (100).

10. The system (100) according to one of the preceding claims,
**characterized in that**
the system (100) further comprises a modular service module (128), preferably microservices, configured to communicate the patient data received from a user interface platform (124) and/or a patient interface (126) to the event management system (104), preferably to the transformation module (114).

11. The system (100) according to one the preceding claims,
**characterized in that**
the system (100) further comprises a data management module (130), preferably data services, configured to aggregate, process, and distribute the patient data across the system (100),
preferably the data management module (130) being configured to communicate with the transformation module (114) and to receive the data events from a data event bus (118).

12. The system (100) according to one of the preceding claims,
**characterized in that**
the system (100) further comprises an interactive interface (132) for medical users for managing medical contents,
preferably the interactive interface (132) being configured to:
- communicate with the medical content management system (122), and/or
- create configurable clinical rules, wherein the rules define how to respond to different types of the patient data (or events), and/or
- create or update recommendation definitions, and/or
- create or update questionnaires.

13. The system (100) according to one the preceding claims,
**characterized in that**
the system (100) further comprises a synchronization control module (134), preferably a fast healthcare interoperability module, configured to synchronize patient data, enable information exchange within the system (100),
wherein the synchronization control module (134) is in communication with a health information system (136) and/or a modular service module (128).

14. A method (200) of supporting healthcare professionals (102), in particular using a system (100) according to one of the preceding claims, wherein the method (200) comprises the steps of:
- transforming (202), using an event management system (102), patient data into a uniform format, wherein the patient data comprises input data received from a patient (106), and/or medical data obtained from healthcare professionals (102) and a health information system (136);
- generating (204), using a transformation module (114), a recommendation in response to receiving the transformed patient data, and
- displaying (206) the generated recommendation on a user interface platform (124).

15. The method (200) of claim 14,
**characterized in that**
the method further comprises:
- reviewing (208) the recommendation, preferably on a user interface platform (124), where the healthcare professionals (102) either approve or disapprove the recommendation;
- optionally, storing (210) the approval / disapproval of the healthcare professional (102) in the modular service module (128) as input for the medical engineer to update the rules;
- optionally, updating (212) medical data within the modular service module (128), preferably changing the treatment plan for the patient;
- optionally, updating (212) medical data within the health information system (136) to include the approved or disapproved recommendation.
